# EUROPEAN PATENT SPECIFICATION

(11) **EP 3 985 679 B1**
(45) Date of publication and mention of the grant of the patent: **23.09.2026**
(21) Application number: 20202570.6
(22) Date of filing: 19.10.2020
(51) Int. Cl.: G16H 15/00, G16H 30/40

(54) **TECHNIQUE FOR PROVIDING AN INTERACTIVE DISPLAY OF A MEDICAL IMAGE**
TECHNIK ZUR BEREITSTELLUNG EINER INTERAKTIVEN ANZEIGE EINES MEDIZINISCHEN BILDES
TECHNIQUE PERMETTANT DE FOURNIR UN AFFICHAGE INTERACTIF D'UNE IMAGE MÉDICALE

(43) Date of publication of application: 20.04.2022
(73) Proprietor: deepc GmbH, 80336 München (DE)
(72) Inventor: Pfister, Franz, 80538 München (DE)
(74) Representative: Laqua, Bernd Christian Kurt

(56) References cited:
- WO-A1-2011/033770
- WO-A1-2013/160382
- CN-A- 110 633 754
- JP-A- 2012 208 726
- JP-B2- 5 263 997
- KR-A- 20190 000 194
- US-A1- 2017 154 156
- US-A1- 2020 205 783

## Description

### Technical Field

The present disclosure generally relates to the field of medical data processing. In particular, a technique for generating a medical report and providing an interactive display of a medical image based on the medical report of a patient is presented. The technique may be embodied in methods, computer programs and apparatuses.

### Background

In the medical field, medical reports of patients that comprise medical findings for the patients are frequently used. Such medical reports shall allow a user (e.g., a doctor or other medical staff) to get a quick overview of a patient's overall or specific medical condition. The user may then decide whether the patient needs treatment, whether a current therapy works or how the patient needs to be treated medically. Consequently, medical reports are critical in the field of medicine for aiding users in their decision-making, thereby directly influencing clinical outcomes.

Currently, medical reports are provided to the user in a printed format. In case the medical report comprises many medical findings, this may result in multi-page documents with a lot of text. Sometimes, decisions of the user may need to be made in relatively short time, such as when an acute medical condition of the patient occurs (e.g., an organ failure or a sepsis). It is evident that the user may not read the complete printed medical report under such enormous time pressure. Even if the user was only interested in certain parts of the medical report, e.g., parts related to certain body parts of the patient, it may be tedious to extract the relevant information from the printed medical report. Still further, correlations between medical findings and body parts of the patients can hardly be visualized on such printed medical reports.

Document US 2020/205783 A1 is related to graphical grading of medical findings during a diagnostic imaging exam. A method includes displaying an acquired medical image on a display area of a display device, displaying a virtual anatomical diagram on the display area adjacent to the ultrasound medical image, responsive to selection of an anatomical region from the anatomical diagram, displaying a plurality of icons representing graded diagnostic findings associated with the anatomical region, and responsive to selection of an icon of the plurality of icons, storing the ultrasound medical image in permanent memory.

Document WO 2011/033770 A1 discloses a radiograph interpretation report creation device that assists in the linking of radiograph interpretation reports and medical images. Said device acquires and displays a medical image, creates and displays an electronic radiograph interpretation report on said medical image, receives as input the location of a lesion region on the medical image, inserts into the radiograph interpretation report, so as to be able to be displayed identifiably, link text linked by a hyperlink with the medical image containing the inputted lesion region location, specifies identifiably-displayed link text on the radiograph interpretation report, and displays on the medical image, in accordance with the specified link text, coordinates representing the location of the lesion region.

CN 110 633 754 A discloses a method for intelligent medical record text recognition based on a neural network. General text and medical record text shall be automatically recognized. Medical words and medical word training data shall increase the predictive ability of medical words.

JP 5 263 997 B2 discusses an approach for creating a medical report by easily and properly specifying auxiliary images which assist a user to understand a key image. A medical report creation device comprises: key image specification means for specifying one tomographic image generated from three-dimensional medical image data acquired by imaging an object as a key image representing diagnostic characteristics; specified position input means for inputting a specified position on the key image; auxiliary image determination means for, based on the key image and the specified position, extracting tomographic images which include the specified position and indicate cross sections different from a cross section of the key image from the three-dimensional medical image data to determine the tomographic images as auxiliary images; and medical report creation means for creating a medical report including information which identifies the key image and the auxiliary image.

US 2017/154156 A1 discusses a radiology workstation including a display device and at least one user input device. A server computer is programmed to operate with the radiology workstation to perform the radiology reading task including a radiology reading support method comprising: receiving user input identifying a radiological finding; retrieving a structured finding object template for the radiological finding; displaying an SFO annotation graphical user interface (GUI) dialog having annotation data entry fields for annotating the retrieved SFO template; building an SFO representing the radiological finding by annotating the SFO template via the SFO GUI dialog; and generating natural language text describing the radiological finding from the SFO. Application program interface (API) action rules may be applied to determine whether the SFO being built satisfies any API action rule, and if so a corresponding application program is invoked.

WO 2013/160382 A1 discusses a system for reviewing a medical image dataset includes a marking storing unit for storing a collection of markings, wherein a marking represents an annotation and a set of viewing parameters in respect of an image dataset. A marking selection unit is arranged for enabling a user to select a marking of the collection of markings for display or insertion. An image display unit is arranged for, in response to the user selecting the marking for display, displaying the image dataset in dependence on the viewing parameters. A marking suggest unit provides a suggestion for information to be added to a marking, in order to better adhere to a reporting standard. Thus, markings can be provided in a structured format allowing for easy review and collaboration.

### Summary

There is a need for a technical implementation which provides an improved display in relation to a medical report of a patient.

Independent method claim 1 defines a method for generating a medical report and providing an interactive display of a medical image based on the medical report of a patient. Independent claims 9 and 10 define an associated apparatus and computer program product, respectively. The dependent claims define specific embodiments.

According to a first example, a method for providing an interactive display of a medical image based on a medical report of a patient is provided. The method comprises a step (a) of displaying a medical image of the patient in a first portion of a display. The method further comprises a step (b) of, in response to a user selecting an indicator visualizing a region of interest, ROI, in the displayed medical image, the ROI associated with a medical finding included in the medical report, displaying a textual representation of the medical finding in a second portion of the display. Alternatively or additionally to step (b), the method comprises a step (c) of displaying a textual representation of a medical finding in a second portion of the display, the medical finding included in the medical report and associated with a region of interest, ROI, in the displayed medical image, and, in response to a user selecting the displayed textual representation, displaying, in the first portion of the display, an indicator visualizing the of the ROI.

This method may display the textual representation in step (b) and the indicator of the region in step (c) in response to a user selection. In other words, the displaying in steps (b) and (c) may be triggered by a user input on a graphical user interface, GUI. Instead of simultaneously displaying all available regions and all indications of medical findings included in the medical report of the patient, the display may react depending on the user selection. This may allow for (e.g., only) the relevant data to be displayed. In other words, a relationship between the region in the medical image and the textual representation of the medical finding associated with the region may be visualized for a user by responsively displaying the related contents upon selection by the user.

The medical image may be an image representing physical or anatomical properties of a body of the patient. The medical image may be a computer tomography, CT, image, a magnetic resonance, MR, image, an x-ray image, a pathological tissue sample image, or the like. The medical image may be an image generated based on a medical measurement of anatomical properties of the patient, e.g., based on an electroencephalography, EEG, measurement, an electrocardiogram, ECG, measurement, blood analysis data, genetic data, or the like.

The first portion and the second portion of the display may be part of the same display unit such as a screen. In another variant, the first portion may be part of a first display unit and the second portion may be part of a second display unit different from the first display unit.

The region may be selected in step (b) by the user by selecting an indicator of the region, the indicator being displayed in the first portion of the display. The region may be selected in step (b) by the user outlining a region within the displayed image. The region may be one-dimensional, two-dimensional or three-dimensional. In other words, the region may be a point, a plane having a certain outline or a volume having a certain outer surface. The region may be a region of interest, ROI. The textual representation may be selected in step (c) by the user clicking on or touching the textual representation displayed in the second portion of the display.

The medical finding may be derived from or be determined based on data describing the body of the patient, e.g., the medical image. The medical finding may be indicative of a medical condition, a medical anomaly, a disease, or the like. The medical finding may comprise a region of interest, ROI, in the medical image or a property of the ROI, such as a size, location, volume, shape, geometry, density, or the like. The medical finding may comprise a property (e.g., of a part, such as the ROI) of the medical image, such as a color, a saturation, a brightness, or the like. The medical finding may comprise physical properties of body tissue of the patient's body, such as a (e.g., optical) density, a type of body cell, or the like. The medical finding may comprise (e.g., an indication of) a medical diagnosis. The medical finding may comprise an identification of an anatomical body part of the patient's body.

The textual representation of the medical finding may be generated based on the medical finding. The medical finding may comprise or be associated to text data comprising or consisting of the textual representation of the medical finding.

The medical report may comprise a plurality of medical findings. The medical report may be a collection of medical findings associated with the patient and/or derived from the medical image of the patient. The medical report may comprise an identifier of the patient.

The indicator of the region may be generated based on the region. The indicator may comprise a visual representation of the region. The indicator may comprise an outline (or "marking") of the region and/or a filling of the region, e.g., using a specific color or visual pattern.

The method may further comprise updating the medical report by adding, in the medical report, a (e.g., first) medical finding based on user input. In other words, the medical report may be configured by the user by adding the medical finding based on user input. This may allow a user to enrich the medical report.

According to a non-claimed example, the medical report may be updated by removing, from the medical report, one or more medical findings based on user input. In other words, the medical report may be configured by the user by removing a medical finding based on user input. This may allow a user to thin out the medical report, e.g., by removing invalid medical findings that no longer match the patient and/or the medical image.

The (e.g., first) added medical finding may be associated with a region defined by the user in the displayed medical image. In this case, the (e.g., first) added medical finding may be determined based on or derived from the region defined by the user in the displayed medical image. The user may define a ROI in the displayed medical image and the (e.g., first) added medical finding may be autonomously (e.g., automatically) determined for the ROI. This may allow the user to specify relevant or interesting regions in the medical image and enrich the medical report with (e.g., first) added medical findings associated thereto.

The method may further comprise displaying, in the first portion of the display, a plurality of indicators of different regions, wherein the (e.g., first) added medical finding may be associated with a group of regions, the group of regions comprising or consisting of a set of the different regions selected by the user. In other words, the user may select the set of the different regions and the (e.g., first) added medical finding may be determined based on the set of the different regions. A same medical finding may be added to the medical report for each of the different regions comprised in the set. Alternatively, or additionally, the (e.g., first) added medical finding may be associated only with the set as such, but not with the individual regions comprised in the set. This may allow a user to group (e.g., only) the relevant or interesting regions into the set and enrich the medical report by adding the (e.g., first) medical finding associated to the set.

The (e.g., first) added medical finding may be defined by the user. The user may define the (e.g., first) added medical finding by selecting the (e.g., first) added medical finding from a plurality of possible (e.g., predetermined, predefined, feasible or available) medical findings. Textual representations of the plurality of possible medical findings may be displayed on the display, e.g., in a list format, to enable the user to select one or more of the possible medical findings. The (e.g., first) added medical finding selected by the user may be assigned or associated to the region defined by the user or to the set of the different regions. The user may define the (e.g., first) added medical finding by inputting a speech command or a textual input. The speech command or the textual input may be converted into or used as the text data of the added medical finding, e.g., as the textual representation of the (e.g., first) added medical finding. This may allow a user to specify the exact (e.g., type or a property of the) (e.g., first) added medical finding to update the medical report.

At least one additional textual representation of a different medical finding associated with (e.g., another region in the medical image or with) the region may be displayed along with the textual representation of the medical finding in the second portion of the display, wherein the different medical finding may be included in the medical report. In other words, the region may have a plurality of associated medical findings, textual representations of which may be simultaneously displayed with the textual representation of the medical finding. This may allow a user to be informed about all medical findings comprised in the medical report that are associated with the region and/or about the region that is associated with the different medical findings represented by the additional textual representations.

A non-claimed example of the method may further comprise, in response to the user designating one or more of the textual representation and the at least one additional textual representation, updating the medical report by removing, from the medical report, any of the (e.g., different) medical findings represented by a textual representation not designated by the user. This may allow a user to choose (e.g., only) the correct and/or relevant (e.g., different) medical findings that are to remain in the medical report. In another non-claimed variant, the method may further comprise, in response to the user designating one or more of the textual representation and the at least one additional textual representation, updating the medical report by removing, from the medical report, any of the (e.g., different) medical findings represented by a textual representation designated by the user. According to this non-claimed variant, a user may choose (e.g., only) the incorrect and/or irrelevant (e.g., different) medical findings that are to be removed from the medical report.

The method may further comprise updating the medical report by adding, in the medical report, a (e.g., second) medical finding, the (e.g., second) medical finding being determined by an artificial intelligence, AI, program module. This (e.g., second) added medical finding may be the (e.g., first) added medical finding described above and/or be determined by the AI module based on the user input (e.g., the region defined by the user in the medical image). The AI program module may be configured to autonomously determine the (e.g., second) added medical finding based on one or more medical findings included in the medical report and, possibly, further based on the user input. Alternatively, or additionally, the AI program module may be configured to autonomously determine the (e.g., second) added medical finding based on the medical image. This may allow for an autonomous or automatic enrichment of the medical report with additional medical findings.

The method may further comprise using at least one of the designated textual representation and the medical finding represented by the designated textual representation as training data for training the AI module. The method may alternatively or additionally comprise using at least one of the (e.g., first and/or second) added medical finding and the textual representation of the (e.g., first and/or second) added medical finding as training data for training the AI module. A machine learning algorithm may be used to provide or improve the AI module based on the training data. The AI module may comprise an artificial neural network, an ensemble of (e.g., AI) program modules, a support vector machine, or the like. This may allow for improving the AI module such that reliability of the (e.g., second) medical findings determined by the AI module can be increased.

At least one of steps (b) and (c) may be repeated after updating the medical report. This may allow for displaying the indicator and/or the textual representation(s) comprised in the updated medical report. In other words, the displayed contents may be updated upon updating the medical report. This may allow informing the user about the current contents of the medical report and make sure that the user is up to date.

The medical finding may be stored as a (e.g., first) node of a graph in a graph database. The (e.g., first) node may have a label matching a type of the medical finding. The (e.g., first) node may have at least one property specifying a detail of the medical finding and/or the textual representation of the medical finding. The region associated with the medical finding may be stored as a (e.g., second) node of the graph. The (e.g., second) node may have a label (e.g., "ROI") matching the region associated with the medical finding. The (e.g., second) node may have at least one property specifying a detail of the region associated with the medical finding and/or the indicator of the region associated with the medical finding. The (e.g., second) node representing the region associated with the medical finding may be connected or linked to the (e.g., first) node representing the medical finding in the graph via an (e.g., first) edge of the graph. The (e.g., first) edge may have a label matching the first and the second node.

The (e.g., first or second) added medical finding may be stored as a (e.g., third) node of a graph in a graph database. The (e.g., third) node may have a label matching a type of the added medical finding. The (e.g., third) node may have at least one property specifying a detail of the added medical finding and/or the textual representation of the added medical finding. The region associated with the added medical finding may be stored as a (e.g., fourth) node of the graph. The (e.g., fourth) node may have a label (e.g., "ROI") matching the region associated with the added medical finding. The (e.g., fourth) node may have at least one property specifying a detail of the region associated with the added medical finding and/or the indicator of the region associated with the added medical finding. The (e.g., fourth) node representing the region associated with the added medical finding may be connected or linked to the (e.g., third) node representing the added medical finding in the graph via an (e.g., second) edge of the graph. The (e.g., second) edge may have a label matching the third and the fourth node.

The medical report may be generated based on the graph. The medical report may be generated based on all nodes of the graph or based on a predefined subset of the nodes of the graph. This may allow for a fast updating of the medical report and for a fast display of the textual representation in step (b) and the indicator of the region in step (c).

According to a second example, an apparatus comprising at least one processor and at least one memory is provided. The at least one memory contains instructions executable by the at least one processor such that the apparatus is operable to perform the method of the first example.

According to a third example, a computer program product is provided. The computer program product comprises program code portions for performing the method of the first example when the computer program product is executed on one or more processors (e.g., the at least one processor of the apparatus of the second example). The computer program product may be stored on one or more computer readable recording media. The computer program product may be carried by a data carrier signal, such as a digital signal stream.

According to a fourth example, a method of generating a medical report of a patient is provided. The method comprises (i) selecting, in response to obtaining medical image-related data of the patient, from a plurality of program modules, at least one program module having an input requirement matching the medical image-related data of the patient, (ii) using the at least one program module selected in step (i) to obtain a medical finding based on the medical image-related data of the patient, (iii) selecting, from the plurality of program modules, at least one program module having an input requirement matching the previously obtained medical finding, (iv) using the at least one program module selected in step (iii) to obtain a medical finding based on the previously obtained medical finding, and (v) generating the medical report of the patient, the medical report comprising at least one of the obtained medical findings.

This may allow for determining a plurality of medical findings based on the medical image-related data of the patient and generating the medical report based thereon. By selecting the appropriate (e.g., matching) program modules in steps (i) and (iii), reliability of the obtained medical findings may be improved. Furthermore, it can be ensured that the selected program modules are capable of providing the medical finding based on the respective input data (e.g., the medical image-related data and/or the previously obtained medical finding). Put in other words, a smooth performance of the method steps (i)-(v) without interruptions due to the selected program modules not being able to provide medical findings may be avoided. One may say that a chain of program modules is generated by selecting the appropriate program modules in steps (i) and (iii), thereby allowing subsequently selected program modules to provide medical findings based on the previously obtained medical findings. That is, the chain of program modules may provide a plurality of medical findings, one being derived from another. This may allow for very specific program modules to be used to improve the individually obtained medical findings. Also, the program modules may be easily replaced with newer or improved versions without negatively affecting the method of the fourth example.

The method of the fourth example may be part of or combined with the method of the first example, and vice versa. In particular, individual steps or all steps of the method of the fourth example may be combined with individual steps or all steps of the method of the first example. For example, the medical report described above for the first example may be the medical report described herein with reference to the fourth example. The (e.g., added) medical finding(s) described above for the first example may be the medical finding(s) described herein with reference to the fourth example.

The medical image-related data of the patient may comprise at least one of a medical image of the patient (e.g., the medical image described above for the first example), a region of interest, ROI, in the medical image of the patient (e.g., the region or ROI described above for the first example), a property of the ROI (e.g., the property described above for the first example) and a medical finding derived from the medical image of the patient (e.g., the medical finding or the added medical finding described above for the first example).

Each of the plurality of program modules may be triggered (or "invoked" / "started") individually. The selected program modules may be triggered responsive to the selection thereof in step (i) or (iii). Step (ii) may be performed in response to the selection of step (i). Step (iv) may be performed in response to step (iii). Each of the plurality of program modules may run on a different computing environment, such as a local computer, a server or a cloud, for example.

The input requirement of the at least one program module may be a property of the at least one program module. The input requirement may be obtained from a provider of the at least one program module. The input requirement may specify a type or content of data that the at least one program module requires (e.g., as input to provide the medical finding). The input requirement may specify a type or content of data that the at least one program module is capable of using as input (e.g., to provide the medical finding). The input requirement may specify a type or content of data that the at least one program module needs to provide the medical finding (e.g., based thereon).

The step (ii) may comprise providing the medical image-related data of the patient to the at least one program module selected in step (i). Step (ii) may comprise triggering execution of or invoking the at least one program module selected in step (i). Step (iv) may comprise providing the previously obtained medical finding (e.g., the medical finding obtained in step (ii)) to the at least one program module selected in step (iii). Step (iv) may comprise triggering execution of or invoking the at least one program module selected in step (iii).

The medical report may be generated in step (iv) based on the medical findings as described above for the first example. The medical report may be generated by including all obtained medical findings in the medical report or by including all medical findings having a certain property or type in the medical report. The medical report may be generated by including a latest of the obtained medical findings in the medical report.

Steps (iii) and (iv) may be repeated at least once. This may allow for a larger number of medical findings to be obtained. Thus, the generated medical report may be enriched with more medical findings. In step (iv), the selected at least one program module may be used to obtain the medical finding based on a plurality of previously obtained medical findings. This may increase the degree of detail or "depth" of the obtained medical findings upon each repetition of steps (iii) and (iv), as these steps upon repetition may use (e.g., a plurality of) the previously determined medical finding(s). Again, it is noted that the medical report used in the method of the first example may be the medical report generated in step (v). Steps (b) and/or (c) may be performed or repeated after step (v). The contents displayed in steps (b) and (c) (e.g., the textual representation(s) and/or the indicator of the region) may be updated responsive to step (v).

Steps (iii) and (iv) may be repeated at least once prior to generating the medical report in step (v). In this case, when repeating step (iii), the previously obtained medical finding mentioned in step (iii) may be the medical finding obtained in step (ii) or the medical finding previously obtained in step (iv) (e.g., obtained before repeating steps (iii) and (iv)). Furthermore, when repeating step (iv), the previously obtained medical finding mentioned in step (iv) may be the obtained medical finding mentioned in step (iii).

Steps (iii), (iv) and (v) may be repeated at least once after generating the medical report in step (v). In this case, when repeating step (iii), the obtained medical finding mentioned in step (iii) may be a medical finding included in a previously generated medical report (e.g., generated in step (v) before repeating steps (iii) and (iv)) and/or the medical finding previously obtained in step (iv). Furthermore, when repeating step (iv), the previously obtained medical finding mentioned in step (iv) may be the obtained medical finding mentioned in (e.g., the repeated) step (iii). After having repeated steps (iii) and (iv), step (v) of generating the medical report may comprise or consist of updating the previously generated medical report.

The at least one program module selected in step (iii) may have an input requirement matching a predetermined subset of the (e.g., previously) obtained medical findings or all of the (e.g. previously) obtained medical findings, e.g., when repeating step (iii). In other words, the input requirement of the at least one program module selected when repeating step (iii) may match some or all of the previously obtained medical findings, e.g., the medical finding obtained in step (ii) and the medical finding previously obtained when performing step (iv). In this case, the at least one program module selected when repeating step (iii) may be used to obtain the medical finding based on (e.g., a predetermined subset of) the medical findings matching the input requirement. This may allow for selecting suitable program modules using a plurality of medical findings as input data. Such program modules may combine several medical findings provided as input and provide the medical finding based on the combination. This may improve reliability or specificity of the obtained medical finding.

Some or all of the selected at least one program module may be used to obtain different types of medical findings. For instance, a first module may be configured to provide an ROI, a second module may be used to obtain an identification of an anatomical body part, and a third program module may be configured to determine an indication of a medical diagnosis. This may allow for obtaining a plurality of different types of medical findings based on the same input data (e.g., based on the medical image-related data in step (ii) or based on the previously obtained medical finding in step (iv)). Thus, the medical report generated in step (v) may include medical findings of different types. This may improve significance of the medical report, enabling a user to more reliably decide on whether and how to treat the patient, in particular when steps (b) and/or (c) of the method of the first example are performed with such a medical report.

One or more of the at least one program module selected in step (i) may be configured to autonomously (e.g., automatically) determine the medical finding based on the obtained medical image-related data, e.g., in response to being selected. Alternatively, or additionally, one or more of the at least one program module selected in step (iii) may be configured to autonomously determine the medical finding based on the previously obtained medical finding, e.g., in response to being selected. The one or more of the at least one selected program module may be an artificial intelligence, AI, module (e.g., the AI module described above for the first example, or an AI module comprising one or more features of the AI module described above for the method of the first example). This may allow for a fast determination of the medical finding.

The method may further comprise, if a plurality of AI modules providing a same type of medical finding are selected in step (i) or (iii), combining the plurality of AI modules in an ensemble and using the ensemble to obtain the medical finding. This may improve reliability of the obtained medical finding.

The medical finding obtained using the ensemble may correspond to the indication of the medical condition determined as described in European patent application EP 20 159 958.6 filed on 28 February 2020. In this case, the plurality of program modules described herein may correspond to the plurality of models described in the aforementioned European patent application. Furthermore, in this case, the selected AI modules described herein may correspond to the selected at least one model described in the aforementioned European patent application.

One or more of the at least one program module selected in step (i) may be user interface, UI, modules that, in step (ii), request a user input defining the medical finding (or, e.g., a region associated with the medical finding). Alternatively, or additionally, one or more of the at least one program module selected in step (iii) may be user interface, UI, modules that, in step (iv), request a user input defining the medical finding (or, e.g., a region associated with the medical finding). Such a UI module may provide the functionality described above for the first example. In particular, such a UI module may perform one or more of steps (a), (b) and (c). The medical finding added based on the user input described for the first example may correspond to the medical finding requested by the UI module. In other words, the "added" medical finding based on the user input as described for the first example may correspond to the medical finding obtained using the UI module. Put in other words, the UI module may be configured to request a user to provide a user input and the UI module may be used to obtain the user input as the medical finding, or to obtain the medical finding by determining the medical finding based on the user input. The medical finding obtained using the UI module may then be included in the medical report in step (v), thereby "adding" the medical finding in the medical report. This may allow a user to influence the medical findings that are used to generate the medical report. In other words, the generated medical report may be improved or updated based on the user input.

If (e.g., only if) the plurality of program modules does not comprise a program module that is configured to autonomously determine the medical finding, the at least one selected program module may be the one or more UI module. In this manner, the obtaining of the medical finding may be ensured by relying on the user input (e.g., defining the medical finding). In this way, a complete medical report may be generated comprising a predefined minimum number of (e.g., types of) medical findings.

The method may further comprise training an (e.g., the) artificial intelligence, AI, module of the plurality of program modules with (e.g., labels and/or properties of) the medical finding(s) obtained using the one or more UI modules. As described above with reference to the first example, this may improve reliability of the medical findings obtained using the AI modules.

The selection of the at least one program module in step (i) and/or (iii) may be performed by an artificial intelligence, AI, selection module. The AI selection module may adapt the input requirements of the program modules or select, from among a plurality of program modules having matching input requirements, at least one preferred module. The AI selection module may select the at least one program module based on an output (e.g., a type of the medical finding) that the program modules provide. The AI selection module may select the program modules providing a preferred output based on (e.g., medical findings included in) a plurality of predetermined medical reports (e.g., generated by a user). This may allow for a selection of program modules providing outputs that comply with the plurality of predetermined medical reports and enable generating consistent medical reports.

The method may further comprise training the AI selection module with some or all of the obtained medical findings. The AI selection module may be trained based on (e.g., the medical findings included in) the plurality of predetermined medical reports. This may allow improving performance of the AI selection module and generating consistent medical reports.

The method of the fourth example may further comprise storing each of the obtained medical findings as a node of a graph in a graph database. The obtained medical findings may each be stored as a separate node of the graph. For example, the medical finding obtained in step (iv) may be stored as a node that is connected to a node representing the previously obtained medical finding used to obtain the medical finding in step (iv). The medical finding obtained in step (ii) may be stored as a node connected to a node representing the image-related data of the patient. In other words, the graph may represent dependencies of the obtained medical findings and/or specify the input data used to obtain the medical findings. One could say that the graph may comprise a chain of nodes representing the obtained medical findings, wherein each node representing an obtained medical finding has been created, generated or determined based on a previous node in the chain. This may allow for fast storing and retrieving the obtained medical findings and for fast and reliably generating the medical report in step (v).

The graph may correspond to the graph described above for the first example. The nodes may correspond to the nodes described above for the first example. Similarly, the nodes may be connected via edges. Each of the obtained medical findings may correspond to one of the medical finding and the (e.g., first or second) added medical finding described above for the first example. The user input used to add a medical finding as described above for the first example may be requested by the UI module to obtain the medical finding. The updating of the medical report described above for the first example may correspond to performing step (ii) or (iv) (e.g., using the UI module) and then performing step (v).

According to a fifth example, an apparatus comprising at least one processor and at least one memory is provided. The at least one memory contains instructions executable by the at least one processor such that the apparatus is operable to perform the method of the fourth example.

According to a sixth example, a computer program product is provided. The computer program product comprises program code portions for performing the method of the fourth example when the computer program product is executed on one or more processors (e.g., the at least one processor of the apparatus of the fifth example). The computer program product may be stored on one or more computer readable recording media. The computer program product may be carried by a data carrier signal, such as a digital signal stream.

Whenever the term "based on" is used herein, this in one variant may mean "based at least on" and in another variant "based only on". When it is referred to "at least one", this may mean "exactly one", "only one" or "two or more".

### Brief Description of the Drawings

Further details and advantages of the technique presented herein will be described with reference to exemplary implementations illustrated in the figures, in which:
- Fig. 1: illustrates an exemplary composition of an apparatus according to the present disclosure;
- Fig. 2: illustrates a method of providing an interactive display of a medical image, which may be performed by the apparatus according to the present disclosure;
- Fig. 3: illustrates a method of generating a medical report of a patient, which may be performed by the apparatus according to the present disclosure;
- Fig. 4: illustrates an exemplary node specification database according to the present disclosure;
- Fig. 5: illustrates an exemplary edge specification database according to the present disclosure;
- Fig. 6: illustrates an exemplary graph according to the present disclosure;
- Fig. 7: illustrates a method and components involved in the method according to the present disclosure;
- Fig. 8a: illustrates a display according to the present disclosure;
- Fig. 8b: illustrates another display according to the present disclosure; and
- Fig. 9: illustrates a method and components involved in the method according to the present disclosure.

### Detailed Description

In the following description, for purposes of explanation and not limitation, specific details are set forth in order to provide a thorough understanding of the present disclosure. It will be apparent to one skilled in the art that the present disclosure may be practiced in other implementations that depart from these specific details.

Those skilled in the art will further appreciate that the steps, services and functions explained herein below may be implemented using individual hardware circuitry, using software functioning in conjunction with a programmed micro-processor or general-purpose computer, using one or more Application Specific Integrated Circuits (ASICs) and/or using one or more Digital Signal Processors (DSPs). It will also be appreciated that when the present disclosure is described in terms of a method, it may also be embodied in one or more processors and one or more memories coupled to the one or more processors, wherein the one or more memories are encoded with one or more programs that perform the steps, services and functions disclosed herein when executed by the one or more processors.

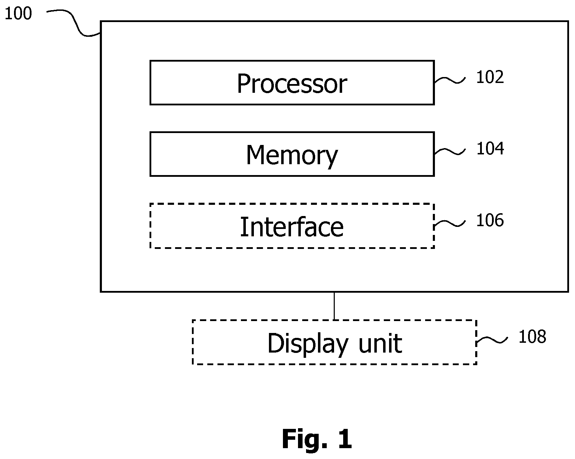

Fig. 1 illustrates an exemplary composition of an apparatus 100 according to the present disclosure. The apparatus 100 comprises a processor 102, a memory 104 and, optionally, an interface 106. The processor 102 is coupled to the memory 104 and, optionally, to the interface 106. The interface 106 is configured to obtain, receive, send or transmit data from or to an external unit such as a data storage unit, a server, a user input unit, an output unit such as a display unit 108, a speaker, or the like. The interface 106 may be configured to send a trigger signal to the output unit to trigger output of an acoustic and/or optical notification (message) for a user. The memory 104 is configured to store a program comprising instructions which, when executed by the processor 102, cause the processor 102 to carry out a method as described herein. The program may be stored on a storage medium. The apparatus 100 may be connected to the display unit 108, e.g., via the interface 106. Alternatively, the display unit 108 may be part of the apparatus 100.

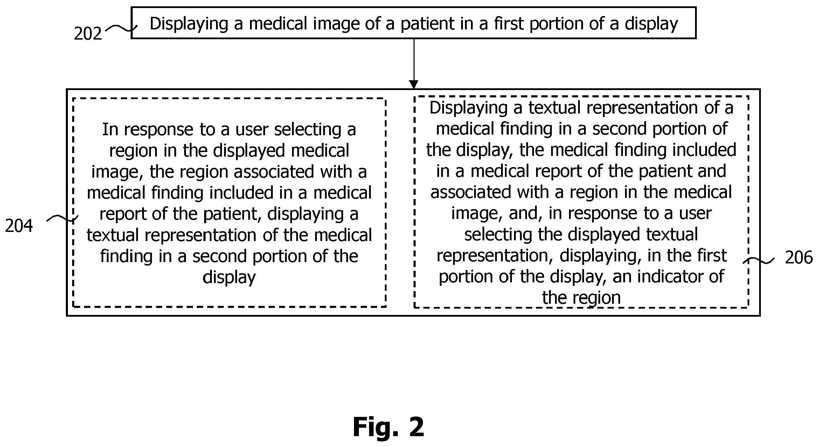

Fig. 2 illustrates an exemplary method in accordance with the present disclosure. The method may be performed by the apparatus 100. The method of Fig. 2 may correspond to the method of the first example described above. The method comprises a step 202 of displaying a medical image of a patient in a first portion of a display, e.g., the display unit 108. The method comprises at least one of steps 204 and 206. In step 204, in response to a user selecting a region in the displayed medical image, the region associated with a medical finding included in a medical report of the patient, a textual representation of the medical finding is displayed in a second portion of the display. In step 206, a textual representation of a medical finding is displayed in a (e.g., the) second portion of the display, the medical finding included in a (e.g., the) medical report of the patient and associated with a region in the medical image, and, in response to a user selecting the displayed textual representation, an indicator of the region is displayed in the first portion of the display. The second portion may be different from the first portion.

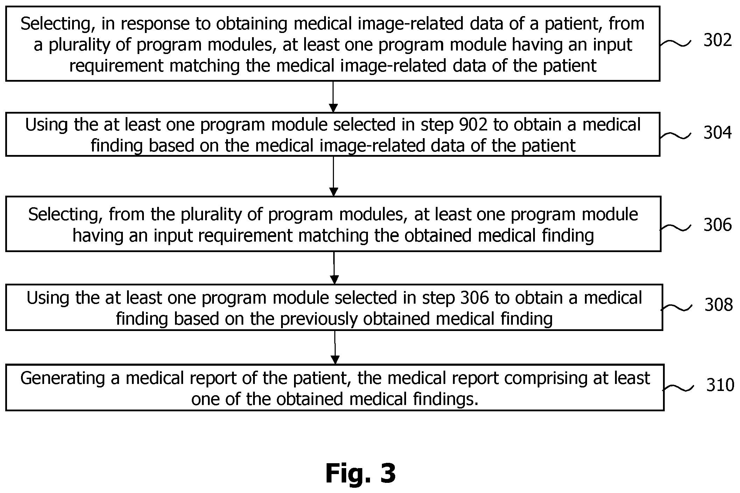

Fig. 3 illustrates an exemplary method in accordance with the present disclosure. The method may be performed by the apparatus 100. The method of Fig. 3 may correspond to the method of the fourth example described above. The method comprises a step 302 of selecting, in response to obtaining medical image-related data of a patient, from a plurality of program modules, at least one program module having an input requirement matching the medical image-related data of the patient. The method further comprises a step 304 of using the at least one program module selected in step 302 to obtain a medical finding based on the medical image-related data of the patient. The method comprises a step 306 of selecting, from the plurality of program modules, at least one program module having an input requirement matching the obtained medical finding. The method further comprises a step 308 of using the at least one program module selected in step 306 to obtain a medical finding based on the previously obtained medical finding. The method comprises a step 310 of generating a medical report of the patient, the medical report comprising at least (e.g., a latest) one of the obtained medical findings.

The method shown in Fig. 2 may be combined with the method shown in Fig. 3. One or more of the steps 202-206 may be performed in combination with one or more of the steps 302-310. When reference is made in the following to "the method", unless stated otherwise or apparent from the subsequent statements, the subsequent explanations may be directed to one or both methods of Figs. 2 and 3 or to a combination of individual steps of these methods forming another method.

Generally speaking, the method described herein can be realized using a graph comprising nodes connected by edges, wherein the graph may be stored in a graph database.

Properties and constraints of the nodes may be stored in a node specification database. In other words, the graph may (e.g., only) comprise nodes that have the properties and fulfill the constraints defined in the node specification database. Properties and constraints of edges may be stored in an edge specification database. In other words, the graph may comprise (e.g., only) edges that have the properties and fulfill the constraints defined in the edge specification database. Properties and constraints for additional nodes may be added to the node specification database and properties and constraints for additional edges may be added to the edge specification database. In other words, the node specification database and the edge specification database may describe conditions that must be fulfilled by the nodes and edges of the graph.

The node specification database and the edge specification database may be part of a same condition database. The node specification database, the edge specification database and the graph database can be implemented in a centralized fashion. One or more of the node specification database, the edge specification database and the graph database may be stored in different locations. The node specification database, the edge specification database and the graph database may be implemented fully decentralized using distributed ledger technology to manage access control, using proof of stake mechanisms, work mechanisms, authority mechanisms, or the like. As an example, a node with a label "CT" may be stored in a patient's database, whereas a node with a label "report" may be stored on a hospital server. The edge connecting the node having the label "CT" with the node having the label "report" can be saved either with edge "CT" or "report" or both to connect the two nodes accordingly.

Each node might comprise or be tagged with a label (e.g. "PATIENT", "CT", "FINDING") that indicates a type of the node. Each node may have at least one property which for example is a key-value-pair (e.g. name:<NAME>"). Each edge may have a label (e.g. ":HAD_AN_EXAMINATION", ":HAS_REPORT") that indicates a type of the edge or a correlation between a start node and an end node connected by the edge. Each edge may comprise at least one property (e.g. weights, costs, or further information). The at least one property of the edge may include metadata about the connection provided by the edge or weights that indicate, e.g., probabilities. Edges may have directions. Directions may indicate that connections are only meaningful along certain directions, e.g., a patient *had an examination* of type CT, but not the other way around.

For example, a node with the label "CT", the node having a computer tomography, CT, image as property, may be connected to a node with the label "DISEASE" via an edge having, as the at least property, the weight indicating a confidence in the correctness of this connection. As another example, a node with the label "PATIENT" may be connected to another node with the label "PATIENT" via an edge having the label "IS_SIMILAR_TO". In this case, a weight assigned to the edge with the label "IS_SIMILAR_TO" might indicate a degree of similarity between two patients represented by the two nodes with the label "PATIENT".

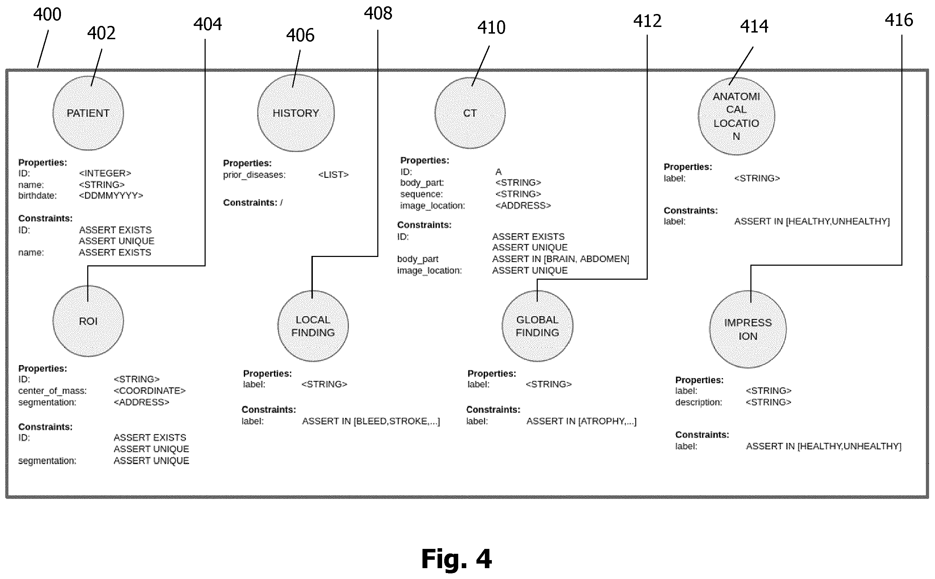

Fig. 4 shows an exemplary node specification database 400 comprising properties and constraints of a plurality of nodes, each node having a unique label 402-416. The properties include a unique identification tag ("ID"), a name of the patient ("name"), a birthday of the patient ("birthdate"), a prior disease ("prior_disease") and so on. The constraints of each node may include constraints for one or more of the properties of the respective node. Constraints may include, but are not limited to, unique node property constraints, node property existence constraints and node property type constraints. As indicated in Fig. 4 for the node with the label 402, the unique node property constraint is for example that the property "ID" in nodes with label "PATIENT" must be unique across all existing nodes with label "PATIENTS". The node property existence constraint may require specific properties to be filled. As indicated in Fig. 4, the node property existence constraint for the node with the label 402 may specify that the field "name" must be filled and must not be left empty. The node property type constraint may require an entry for a specific property of a node to be of a certain type (e.g. a string, an integer, a date, or an address to a data storage location).

In sum, the node specification database 400 may contain all possible nodes that can be filled with information. Each element in the node database may describe a node and particularly at least one of a label (e.g., "PATIENT", "CT", "REPORT") indicating a type of the node, a list of properties associated with the type of the node (e.g., key-value pairs like "ID:<STRING>", "name: <STRING>"", "birthdate:<DDMMYYYY>"), and constraints associated with that node type.

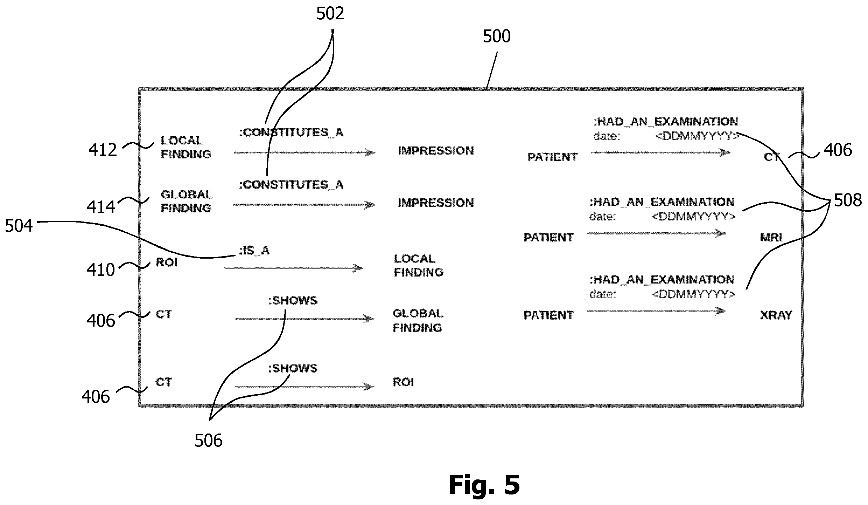

Fig. 5 shows an exemplary edge specification database 500 comprising definitions of a plurality of edges, each edge having a unique label 502-516. The edge specification database 500 may comprise edge property existence constraints and/or edge property type constraints, as similarly described above for the nodes of node specification database 400. The edge specification database 500 may comprise edge assignment constraints defining a label or type of a start node (and, e.g., a label or type of the end node). An edge assignment constraint may require an edge with a specific label to only connect nodes of specific types, e.g., an edge with label ":HAD_AN_EXAMINATON" may only connect a start node of type "PATIENT" with a target node "CT", "MR", etc. A further edge assignment constraint might be that it can only connect one source (e.g., start) node with one target (e.g., end) node, and not one source node with many target nodes or many source nodes with the same target node. In other words, the edge specification database 500 may define a possible type of start node and a possible type of end node for each edge. For example, the edge having the label 502 may only connect a node having the label 412 as the start node and a node having the label 416 as the end node, and the edge having the label 508 may only connect a node having the label 406 with a node having the label 414 as the end node and so on. The edge specification database 500 may comprise properties of the edges, as indicated for the edges 512-516 having the property "date" defining a date when (e.g., a property of) the end node was created.

In sum, the edge specification database 500 may contain all possible edges that can be used to link certain types of nodes. Each element in the edge specification database may describe an edge and particularly at least one of a label (e.g. "HAD_AN_EXAMINATION", "IS_BASED_ON") indicating a type of the edge, a list of properties associated with the type of the edge (e.g. key-value pairs like "date:<DDMMYYYY>"), a type of the input node (e.g., start node) the edge requires, a type of the output node (e.g., end node) the edge requires, and constraints associated with that edge type.

Note that in the example shown in Fig. 5, the edge ":HAD_AN_EXAMINATION" may have a constraint that a patient can have multiple CTs (1: many), but a single CT cannot be assigned to multiple patients. Multiple regions of interest (ROI) may be associated with a same local finding (many:1). Multiple nodes of type local or global finding may point to an impression (many-of-different-type:1).

The nodes and edges of the graph may be fully defined by the types of nodes specified in the node database 400 and the types of edges specified in the edge database 500. The node database 400 and the edge database 500 might be (e.g., dynamically) modified by amending their contents, e.g., by adding additional types of nodes or additional edges. A user may remove certain (e.g., types of) nodes, edges, properties and constraints in the databases 400 and/or 500, and replace them by others. Editing of the databases 400 and 500 may be performed via a graphical user interface (GUI). Different person groups might have different access rights to change the contents of the databases 400 and 500.

The databases 400 and 500 may be queried. Commands may be "LIST_ALL_EDGES WITH SOURCE NODE of type "PATIENT"" or "LIST_ALL_EDGES WITH TARGET NODE of type "FINDING"". Such a query may result in a generation of a graph representing a medical report. A corresponding graph may only be generated or created if the queries are valid with regard to the node specification database 400 and the edge specification database 500.

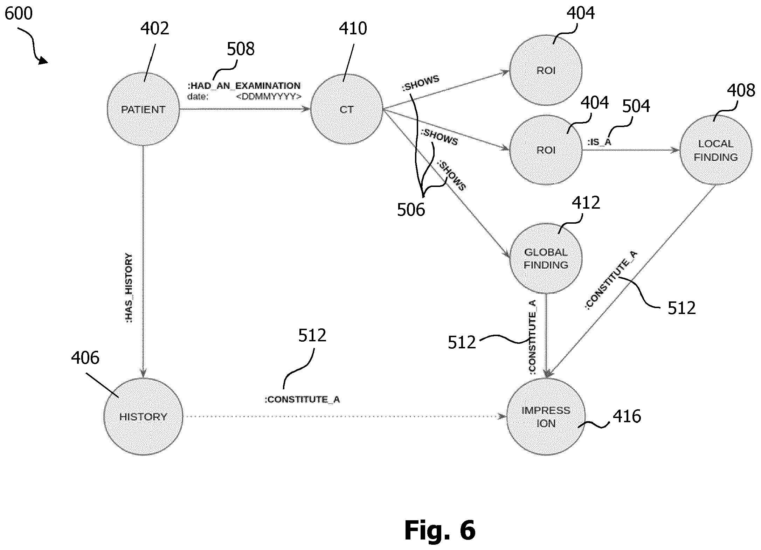

Fig. 6 shows an example of a graph 600 generated using the node specification database 400 and the edge specification database 500. The nodes of the graph 600 have the properties and fulfill the constraints defined by the node specification database 400. The edges of the graph 600 comply with the edge definitions stored in the edge specification database 500.

In this example, for a patient, CT image data was acquired. The CT image data may comprise a medical image that may show two regions of interest (ROI), where only one may turn out to be a local finding and the other one may be regarded as being normal and may not be associated with a local finding. Furthermore, the patient may show a global finding, like atrophy, for example. Together, the local and the global finding as well as the patient history may constitute an impression (e.g., "the patient is not healthy, suffers from disease UVW and therapy XYZ is recommended").

Program modules may be provided that may infer new parts of the graph 600 based on other parts of the graph 600. In other words, the program modules may use certain portions of an existing graph and determine new nodes to be included in or added to the graph. The modules can take some or all properties of an input node to determine or provide an output, e.g., create an output node. The modules may be stored in a module database. The modules may be triggered fully automatically, whenever a required information (e.g., a required node) is available in the graph 600. The modules may alternatively be triggered by a user interaction or by a meta-model-logic, also referred to as AI selection module herein. A program module may be a user interface, UI, module, enabling a human to provide user input, an artificial intelligence, AI, module, an ensemble of AI modules, or another software product autonomously producing outputs. The program module may be a database entry of the graph database. The program module may run on an external system that is able to communicate with the apparatus 100, e.g., via the interface 106.

Each program module may have input requirements specifying the types of the input node(s) it needs. Each program module may specify the output it produces, e.g., a type of node it creates. A module can have different output types: Modules that output a single filled node (e.g., a node having a label and also a property) without an edge connection, and modules that output a filled node plus a connection that specifies how the filled node is appended to the input node. The modules may specify additional constraints, e.g., a module might only be able to produce the specified output based on a specific property of the input node. For example, a module might only be usable if an input node of the type "PATIENT" has a property "sex="FEMALE"". Outputs that are produced by a program module may be automatically appended to the existing graph in case their output complies with the node specification database 400 and the edge specification database 500, particularly with the constraints specified therein. Otherwise, the program module may return a warning.

A program module might perform a database query. Examples for such databases that might be queried by a module may include a Radiological Information System (RIS) and a Picture Archiving and Communication System (PACS).

For example, if a node with the label "PATIENT" is created, a program module (e.g., named or labeled "QUERY_FROM_RIS") may query a patient history from the RIS system. The program module may perform some further processing or aggregation, create a node with the label "HISTORY" and an edge with the label "HAS_HISTORY"" and append the same to the node with the label "PATIENT", thereby adding a new node and edge to the graph.

As another example, if a node with the label "PATIENT" is created, a program module (e.g., named or labeled "QUERY_FROM_CT _FROM_PACS") may query a last CT exam taken for the patient with the respective ID from the PACS system, store it in an object storage, output a node with the label "CT", and add the address to this object storage to the property "ADDRESS" of the output node.

A module may represent a user interface, UI, or graphical user interface, GUI, module to query information from a user.

A simple example may be a text field that may be displayed (e.g., on the display 108) along with a CT examination (e.g., a CT image of the patient). In case text is filled into the text field by a user, this text may be appended to the node labeled "CT" as a property of a node labeled "IMAGE ANALYSIS RESULTS" connected to the node labeled "CT" via an edge.

Another example may be a program module that specifies a CT image as an input requirement. The module may display the image within a viewer (e.g., in the first portion of the display unit 108) and query a click from the user. The coordinates of the click may be appended as properties of a node labeled "ROI" to the node labeled "CT" via an edge labeled ":SHOWS" (see also Fig. 6).

Another example may be a program module that specifies a CT image and multiple regions of interests (e.g., regions comprising multiple sclerosis, MS, lesions) as input requirements. The module may display (e.g., in the first portion of the display unit 108) the CT image within a viewer along with the regions of interests as overlay maps, and query the user to mark multiple of those regions of interest and group them to a "FINDING". The "FINDING" may be the label of a node in the graph, connections of which may be added or modified based on the grouping of the user, e.g., by linking several nodes labeled "ROI", corresponding to the marked ROIs, with the node labeled "FINDING".

Another user interaction may be speech. For example, a module may define provision of a CT image as an input requirement. The module may query the user to formulate his impression (e.g. "THE USER IS HEALTHY") and transform it (e.g., via natural language processing, NLP) into a filled node and edge, and append the same to the graph.

Modules may perform algorithmic operations to produce outputs, e.g., based on machine learning algorithms.

For example, a module might require a node labeled "CT" as input. The module may run a general anomaly detector and output multiple regions of interest, and append each ROI as a separate node labeled "ROI" to the node labeled "CT". The anomaly detector may be trained beforehand.

As another example, a module (e.g., labeled "ATLAS_AI") may require a node labeled "ROI" as input, determine an anatomical location of a finding via atlas matching based on the properties of the node labeled "ROI", and add the location as a property of an additional node labeled "LOC" to the node "ROI".

There may be modules that combine several types of modules, e.g. a module may perform a database query in a first step, and then perform an algorithmic computation based on the queried information to fill or add a node of the graph. As another example, a module may perform an algorithmic computation based on a CT image, and then request confirmation of a determined ROI by the user via the user interface to fill or add a node of the graph.

As noted above, the modules may be stored in the module database. The module database may be queried itself, e.g., for a given input node, all modules that require this type of input node can be output. For example, a query for the input node "CT" could return the following modules: "PERFORM_ANOMALY_DETECTION", which may be an algorithmic module; "PERFORM_BLEED_SEGMENTATION", which may be an algorithmic module; and "USER_ANOMALY_SEGMENTATION", which may be a UI module, requesting the user to segment an anomaly in the CT image. As noted above, the CT image may be included as a property of the node "CT" or linked to the node "CT" by a property of the node "CT".

After every change of the graph, for example upon adding an additional node, a query may be made to the module database. Based on the current status of the graph, a query may be made and the modules that are available may be returned. In other words, at least one of the modules may be selected that has an input requirement matching (e.g., at least) the added additional node. The input requirement may specify a plurality of node types that are needed. The query of the module database may return all modules that have input requirements matching a subgraph of the current graph. Each returned or selected module may contain the information about the way it is triggered. For such a query, a list of modules that are available may be returned, and depending on their respective module type (e.g., UI module, AI module, other algorithmic module) they may be triggered differently.

That is, the selected (e.g., returned) modules may be triggered in different ways. Options include automatic triggering, triggering via a meta-model and triggering via a user interaction.

Modules may be triggered automatically as soon as the inputs they require are available in the graph. For example, if an algorithmic module that is configured to perform general anomaly detection in a CT image requires a node labeled "CT" having a CT image as the property, the module may be triggered automatically.

A module may be triggered via a logic in a meta-model (e.g., via an AI selection module). By using existing data, e.g., a plurality of previously generated graphs, a machine learning meta-model may be trained that learns to recommend specific modules, and may automatically trigger them. For example, a meta-model may have learned based on existing data that it should always run an "Alzheimer" detector module for patients older than 70 years, while for younger patients this model may be omitted.

Modules may be triggered via a user interaction. As an example, if a user draws a rectangle in a CT image to define a region (e.g., ROI), an algorithmic module may be triggered that searches for anomalies in the specific region. A simple example for a trigger via a user interaction may be a simple button that is clicked by a user.

Another relevant type of user interaction may be an "Autofill"-functionality. For example, if a user hovers over a highlighted anomaly that may display the inputs of a node labeled "ROI", a module database query might be performed. All modules that require an input node labeled "ROI" may be listed and displayed to a user. Examples of such modules may include: "COMPUTE_SIZE", a module that may be configured to compute a size of the ROI; "SPECIFY_FINDING", a GUI module that may requests the user to specify different types of diseases connected to this finding via a drop-down menu; and "COMPUTE_LOCATION", an algorithmic module that may be configured to compute the location of the finding by atlas matching. In other words, the selection of the at least one program module may be performed by a user, possible based on a list of program modules having input requirements matching a region specified by the user.

A single node (e.g., labeled "CT") might trigger a cascade of modules. For example, once a single node labeled "CT" is created, this may automatically trigger a general anomaly detection module. After the general anomaly detection module has output one or more ROIs and respective nodes labeled "ROI" have been added to the graph connected to the node labeled "CT", another module may compute anatomical locations of the one or more ROIs. Each anatomical location may then be added as an additional node labeled "LOC" and connected to the respective node labeled "ROI". For each of the one or more ROIs, their anatomical location, and a patient history node labeled "HISTORY", an algorithmic module may compute a list of possible diagnoses and append a node labeled "DIAGNOSIS_LIST" comprising the list as a property. This node may be connected to the respective node(s) labeled "ROI", "LOC" and "HISTORY". A UI module may in response to the generation of the node labeled "DIAGNOSIS_LIST" display a textual representation of the node labeled "DIAGNOSIS_LIST" via a GUI, e.g., by displaying each of the possible diagnoses in the list in text format in the second portion of the display 108. Subsequently, a radiologist may select one of the textual representations to confirm a diagnosis, and a corresponding node labeled "DIAGNOSIS" may be added to the graph.

The filled graph or the medical report generated based thereon may be displayed via a UI, e.g., the GUI. This may happen dynamically and step by step as soon as more and more information is filled in the graph.

As an example, if a radiologist clicks on a patient entry, a viewer may open and a "PACS_QUERY" module, as well as a "RIS_QUERY" module, may be triggered. As soon as the "PACS_QUERY" module returns a last CT image from a patient, this image may be displayed in the viewer (e.g., in the first portion of the display 108). As soon as the "RIS_QUERY" module returns the patient history from the RIS and the respective node labeled "HISTORY" is created, the patient history may be displayed via text next to the CT image (e.g., in the second portion of the display 108). As soon as an anomaly detection module returns ROIs as output and the respective nodes labeled "ROI" are created, each of the respective ROIs may be displayed as an overlay to the original image by way of the indicator.

Not all information contained in the graph may be displayed via the GUI. For example, in the beginning, the module database may comprise a UI module (e.g., requesting a user to select a diagnosis) and an algorithmic module (e.g., comprising a machine learning algorithm configured to predict a diagnosis) based on a CT image. Both modules may be triggered whenever a CT image arrives (e.g., whenever a node labeled "CT" is added to the graph), but the output of the algorithmic module may not be displayed to the user. The two nodes created by the algorithmic module and the UI module as output may be appended to the graph database. This data may be used for further analysis, e.g. to monitor the performance of an algorithm before it is deployed in practice. This may also be referred to as a "ghost mode". One may say that the medical report in this case may be generated based on a subset of the nodes of the graph.

The graph, that may be continuously filled, for example, may be used to perform continual learning and improve existing algorithmic (e.g., AI) modules.

The graph and/or the graph database may be used to create new algorithmic modules. For example, in the first place, there might be a "DISEASE_SELECTION" UI module that is used by the radiologist. The outputs provided by this module may be used in connection with the input CT image(s) to train an algorithm that performs a disease classification based on images. Such an approach may enable replacing any of the UI modules (e.g., step by step) with one or more algorithmic modules.

The graph may be used to train meta-models (e.g., the AI selection module) on how to trigger program modules. The graph may be used for statistical analyses (e.g., inferring population statistics or similarity search for patients). The graph data may be transformed into one or more different output formats, e.g., a written medical report, speech, etc. Parts of the graph may be written back to other databases, e.g. to the RIS.

Some of the nodes described herein (e.g., the nodes having the label "ROI", "LOCAL FINDING", "GLOBAL FINDING", "ANATOMICAL LOCATION" or "IMPRESSION") may also be referred to as "medical findings". In other words, the graph may comprise a plurality of medical findings. The medical report may thus be generated based on the graph by including one or more of the medical findings of the graph in the medical report of the patient. The medical report may be a collection of nodes of the graph. The medical report may be generated by selecting several nodes from the graph and generating a dataset or file including (e.g., links to) the properties of the selected nodes. The nodes may be selected based on their labels, e.g., using a predetermined list of labels to be selected. The nodes "CT" and "ROI" of the graph 600 are examples of the medical image-related data of the patient. The textual representation of a medical finding may correspond to or be based on a property of the node representing the medical finding (e.g., the property "label" of the node with the label "LOCAL FINDING" shown in Fig. 4).

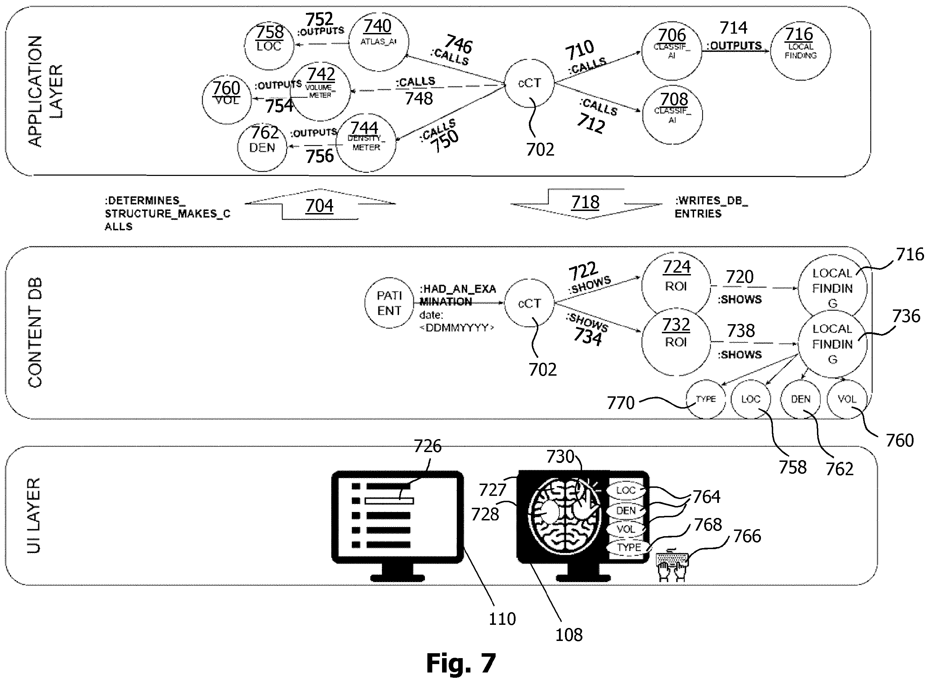

Fig. 7 shows a schematic illustration of a method and components involved in the method in accordance with the present disclosure. The method may comprise some or all of the method steps 202-206 and 302-310. The method may be performed by the apparatus 100. The method steps may be executed in one of three domains: either on a (e.g., graphical) user interface, denoted as "UI LAYER", in a content database denoted as "CONTENT DB" or in an application layer denoted as "APPLICATION LAYER". The content database may comprise the graph database. The content database may further comprise the node specification database and/or the edge specification database. The content database may be the graph database.

A computed CT image cCT may be obtained, e.g., from the PACS or by loading the image based on a user command. A node 702 labeled "cCT" may be created in the content database and connected to the node "PATIENT" over an edge complying with the constraints defined in the edge specification database 500, thereby extending the graph. The cCT image may be included as a property of the node labeled "cCT" or linked to the node 702 by a property of the node 702 (e.g., a property "address of image location"). In the next step, at least one of the program modules stored in the module database may be selected. To this end, the (e.g., properties of) node 702 may be provided to the application layer in step 704. The selected at least one program module 706, 708 may have an input requirement matching the node 702. Each of the selected at least one program module may be used to obtain a different medical finding in steps 710, 712. In the given example, each of the selected at least one program modules 706, 708 may be called in steps 710, 712. The module 706 may provide a "LOCAL FINDING" 716 as medical finding in step 714, based on the input.

In step 718, the medical finding may be written into the content database. This may result in the graph to be updated by including the medical finding as an additional node 716. In the given example, the updated graph contains the node 716 connected to the node 702 via edges 720, 722 and a node 724 labeled "ROI". The node 724 is connected to the node 716 via the edge 720 and connected to the node 702 via the edge 722. Put in other words, the medical finding represented by node labeled "LOCAL FINDING" is associated to the region represented by the node labeled "ROI", and the region represented by the node labeled "ROI" is associated to the medical image of the node labeled "cCT". That is, a medical finding stored as a first node of the graph may be associated to a region such as a ROI stored as a second node of the graph by an edge connecting the first node with the second node. The region may itself be a medical finding. A medical finding stored as a first node of the graph may be associated to another medical finding stored as a second or third node of the graph by an edge connecting the first node with the second or third node. Each of these nodes and edges may be determined by the selected at least one program module 706 in step 714.

A medical report may be generated based on the updated graph, including the newly added medical findings. A textual representation 726 of one or more of the medical findings comprised in the medical report may be displayed on the second part of a display. Upon clicking on the textual representation 726, an indicator 728 of the ROI represented by the node 724 may be displayed in the cCT image 727 in the first part of the display. Alternatively, or additionally, the textual representation 726 may be displayed upon the user clicking on the indicator 728. In this example, the first portion and the second portion of the display may be part of separate display units 108, 110 comprised in or connected to the apparatus 100.

The user may define another region in the medical image in step 730. In response thereto, a new node "ROI" 732 complying with the requirements set out in the node specification database 400 may be added to the graph, connected to the node 702 via an edge 734 complying with the constraints defined in the edge specification database 500. The user may also define another medical finding such as a "LOCAL FINDING" associated to the ROI. The node 736 having the label "LOCAL FINDING" may be appended to the graph via a suitable edge 738 connecting the nodes 736 and 732. Creation of the node 736 may trigger another selection of at least one program module, this time having an input requirement matching the newly added node 736. In the shown example, the program modules 740-744 "ATLAS_AI", "VOLUME_METER" and "DENSITY_METER" are selected. Each of these selected modules 740-744 may, in response to being selected, provide a (e.g., different) medical finding. In the shown example, each of the modules may be called in steps 746-750 and provided with the node 736 and/or the node 732 as input data. The modules 742-744 output, in steps 752-756, a location, volume and density based on the input data.

Again, as in step 718, these medical findings obtained in steps 752-756 are written to the graph database as nodes 758-762. In the given example, the location, volume and density are each added as additional medical findings to the graph, as illustrated with the nodes 748-752. Again, the medical report may be updated based on the newly added nodes. In the shown example, textual representations 764 of the newly included medical findings may be displayed to the user on (e.g., the second portion of) the display.

That is, the step 730 may trigger a selection of suitable program modules, and trigger the selected modules to output medical findings based on the user input of step 730. The medical report may then be updated by including the so determined medical findings and a visualization of the medical report may be provided to the user. The user may again "jump" from one medical finding to another by selecting the textual representation thereof, whilst being informed of the region associated to the selected medical finding by the dynamically displayed indicator of the selected region. Similarly, the user may select one of a plurality of regions in the medical image and be provided with textual representations of all medical findings associated with the selected region, the textual representations being generated based on or included in the medical report.

In step 766, the user may provide a user input in text format. The user input may define a medical finding. The information input by the user may be displayed on the display (758) and then stored as (e.g., a property of) an additional node 770 in the graph database. The medical report may then once again be updated, e.g., by including the text input as an additional medical finding. The steps 730 and/or 766 may be performed upon request of a UI module.

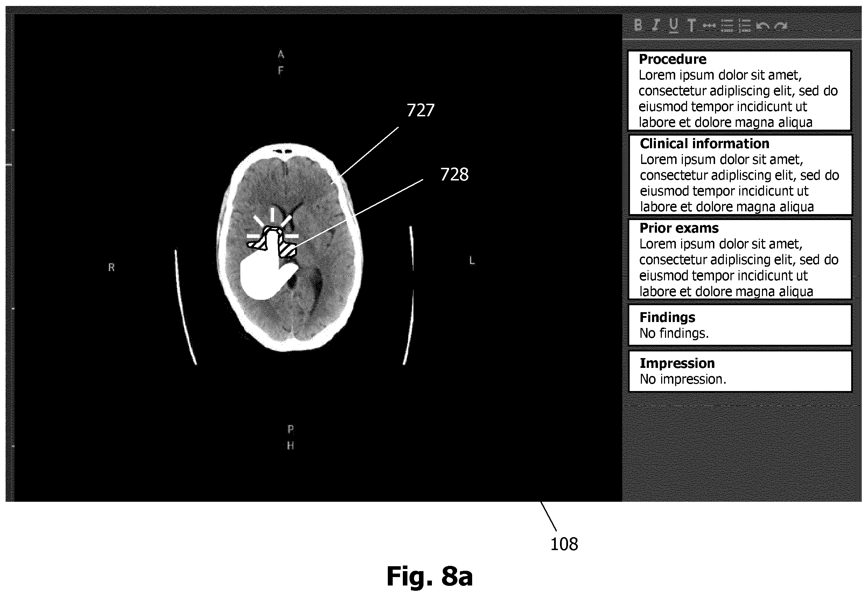

Fig. 8a shows an exemplary display in accordance with the present disclosure. The display may be shown by the display unit 108. The display may correspond to the example indicated in the bottom portion of Fig. 7. As can be seen, the medical image 727 may be displayed in the first portion of the display unit 108. The indicator 728 of the ROI represented by the node 724 may also be displayed in the first portion. In the second portion of the display unit 108 indicated on the right side in Fig. 8a, a plurality of textual representations of medical findings may be displayed. In the shown example, only a procedure, clinical information and prior exams may be displayed in a text format. Then, upon a user selecting the exemplary indicator 728 (as indicated with the hand symbol in Fig. 8a), a textual representation of a medical finding associated to the region represented by the node 724 and visualized by the indicator 728 may be displayed. For example, a textual representation of the local finding represented by the node 716 may be displayed on the second portion of the display in response to the user selecting the region visualized by the indicator 728. In the shown example, the textual representation of the medical finding may be displayed under a title representative of the type of the medical finding (e.g., the title "Findings" or "Impression" indicated on the right side of Fig. 8a; in the shown example, such textual representation is not yet shown and the display thus still indicates "No Finding" or "No Impression").

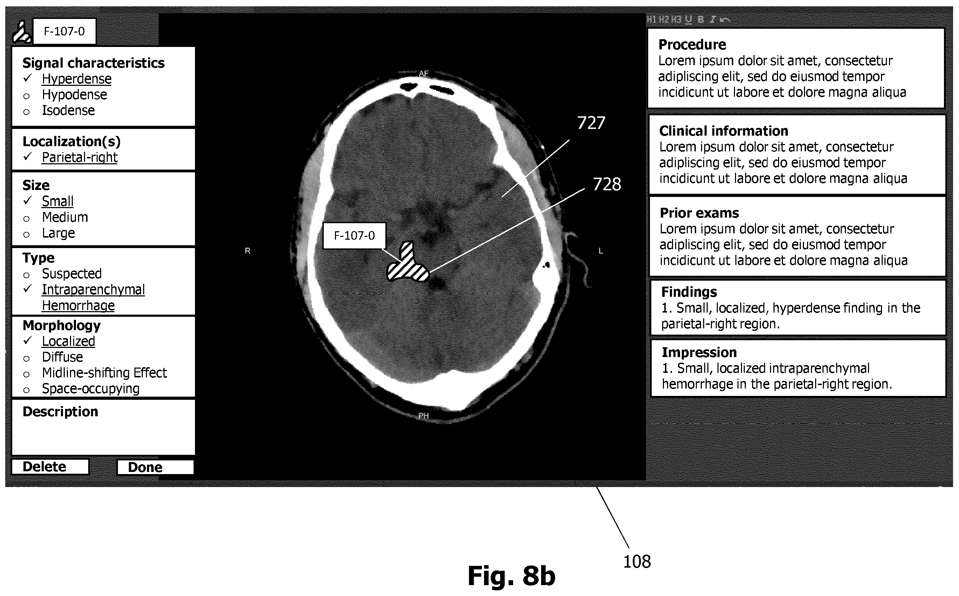

Fig. 8b shows an exemplary display after the user has clicked on the indicator 728 in as described above for Fig. 8a. It can be seen that under each of the titles "Findings" and "Impression", a textual representation of a medical finding may be displayed. The textual representation may be a representation of a medical finding associated to the region represented by the node 724 and visualized by the indicator 728 previously selected by the user. The textual representation displayed may be a textual representation of the local finding represented by the node 716. The user may modify or add (e.g., the) medical findings and/or (e.g., the displayed) textual representations. For example, the user may define properties of a medical finding (e.g., properties of the node representing the medical finding) by selecting at least one property from a list of available properties, as indicated on the left side of Fig. 8b. In case the indicator 728 was previously selected, any newly added medical findings may be associated to the region visualized by the indicator 728. This may enable the user to "enrich" a selected ROI in the medical image 727 with medical findings.

In another variant, in which a selection of a textual representation is made rather than a selection of an indicator on the medical image, the textual representation of the medical finding represented by the node 716 may be displayed in the second portion of the display unit 108, e.g., under the title "Findings", and the medical image 727 may be displayed in the first portion without the indicator 728 at first. Additional textual representations of different medical findings may also be displayed in the second portion, e.g., the textual representations 764 and/or 768. Then, in response to a user selecting the textual representation of the medical finding represented by the node 716, the indicator 728 may be displayed in the medical image 727 in the first portion of the display unit 108.

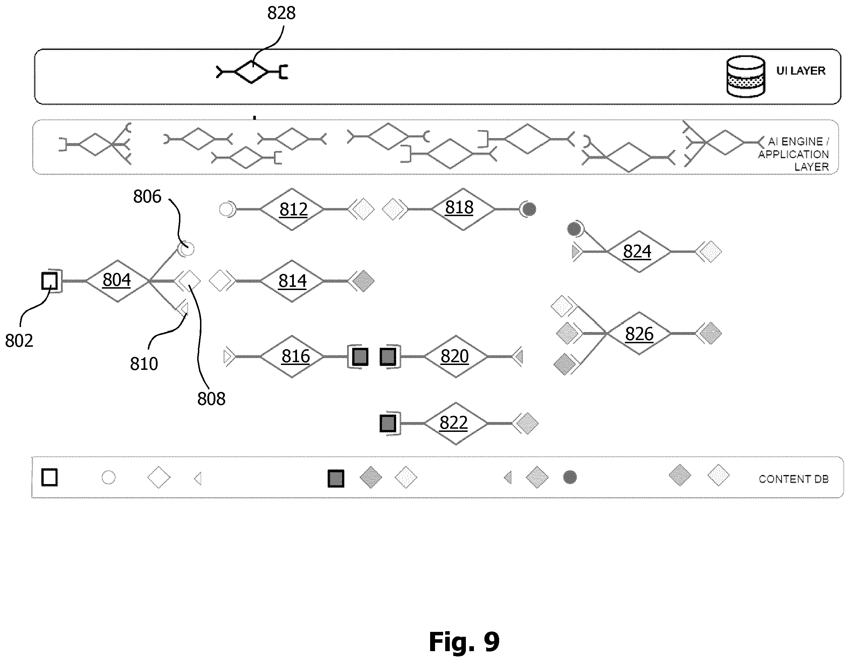

Fig. 9 shows a schematic illustration of an exemplary method and components involved in the method in accordance with the present disclosure. The method may comprise some or all of the method steps 202-206, 302-310 and the steps described above with reference to Figs. 7 and 8. The method may be performed by the apparatus 100. The method steps may be executed in one of three domains: either on a (e.g., graphical) user interface, denoted as "UI LAYER", in a content database denoted as "CONTENT DB" or in an application layer denoted as "AI ENGINE / APPLICATION LAYER". The content database may comprise the graph database. The content database may further comprise the node specification database and/or the edge specification database. The content database may be the graph database. The layers may correspond to the layers shown in Fig. 7.

The program modules may be stored in the module database and perform functions in the "AI ENGINE/APPLICATION LAYER". Medical image-related data 802, such as the cCT, may be obtained. A suitable program module 804 having an input requirement matching the data 802 may be selected and used to obtain medical findings. The medical findings may be of different types, e.g., of a first type 806 (e.g., a ROI), a second type 808 (e.g., a LOCAL FINDING) and a third type 810 (e.g., a volume of the ROI). The medical findings may be written into the content database. In other words, the graph may be extended with new nodes corresponding to the medical findings obtained using the module 804. The medical report may then be updated based on the extended graph.

Next, program modules having an input requirement matching one of the first to third type 806-810 may be selected. In the shown example, these selected modules are the modules 812-816. Each of these modules may again provide different medical findings based on the medical findings previously determined using the module 804. A type of the medical findings provided by the modules 812-816 may differ from one another. Also, in this case, after having determined new medical findings using the modules 812-816, the graph and the medical report may be updated. One further iteration of this procedure is illustrated in Fig. 9 for selected modules 818-822. The selected modules may have an input requirement matching more than one previously obtained medical finding, as illustrated for the modules 824 and 826. The modules 804, 812-826 may comprise or correspond to the modules 706, 708, 740-744 described above with reference to Fig. 7.

A UI module 828 may be provided having an input requirement matching a certain (e.g., type of) medical finding. The UI module 828 may rely on user input to provide a medical finding. For example, the UI module 828 may request a user to provide the user input as described above for step 730 or step 766.

All obtained medical findings may be stored (e.g., as nodes of the graph) in the content database. The medical report may be generated, comprising at least a latest of the obtained medical findings, e.g., all of the obtained medical findings stored in the content database.

## Claims

1. A method for generating a medical report and providing an interactive display of a medical image (727) based on the medical report of a patient, the method comprising:
obtaining a medical image (cCT);
creating a medical image node (702) in a graph stored in a graph database using the medical image, wherein the medical image node (702) comprises the medical image as a property, and wherein the medical image is one of: a computed tomography, CT, image, a magnetic resonance, MR, image, an x-ray image, or a pathological tissue sample image;
automatically triggering an anomaly detector module configured to define a region of interest, ROI, labeled node (724) in the graph, wherein the ROI labeled node (724) is connected to the medical image node (702);
obtaining, for the ROI labeled node (724), a medical finding using a program module selected from a program module database,
storing the medical finding as a local finding labeled node (716) of the graph, wherein the medical finding of the local finding labeled node (716) is associated to an ROI represented by the ROI labeled node of the graph by an edge connecting the local finding labeled node and the ROI labeled node;
associating the ROI node with its corresponding medical finding node via an edge; and
generating the medical report based on the graph comprising a textual representation of one or more of the medical findings;
wherein the method further comprises:
(a) displaying (202) a medical image (727) of the patient in a first portion of a display;
the method further comprising at least one of (b) or (c):
(b) in response to a user selecting an indicator (728) visualizing the ROI stored as the ROI labeled node (724) of the graph in the displayed medical image (727), the ROI associated with the medical finding included in the medical report, displaying (204) a textual representation (726; 764) of the medical finding stored as the local finding labeled node (716) of the graph in a second portion of the display;
(c) displaying (206) a textual representation (726; 764) of the medical finding stored as the local finding labeled node (716) of the graph in a second portion of the display, the medical finding included in the medical report and associated with the ROI stored as the ROI labeled node (724) of the graph in the displayed medical image (727), and, in response to a user selecting the displayed textual representation (726; 764), displaying, in the first portion of the display, an indicator (728) visualizing the ROI;
wherein the method further comprises:
updating the medical report (i) by adding, in the medical report, a medical finding based on user input, wherein the added medical finding is associated with a region defined by the user in the displayed medical image, or (ii) by updating the medical report by adding, in the medical report, a medical finding, the medical finding being determined by an artificial intelligence, AI, program module,
defining, based on the user input, a further ROI of the medical image as a further ROI node of the graph and an added medical finding associated to the further ROI as a further local finding node of the graph, wherein creation of the further local finding node triggers a selection of a program module having an input requirement matching the further local finding node;
updating the medical report based on the further ROI node and the further local finding node;
wherein, upon addition of the further ROI node and the further local finding node to the graph, the method further comprises:
performing a query of the program module database;
obtaining one or more program modules based on the current status of the graph;
triggering the one or more program modules, wherein the one or more program modules are at least one of:
an image viewer configured to display medical image-related data;
an anomaly detection module configured to generate a region node;
a localization module configured to generate an anatomical location node;
a diagnosis module configured to generate a diagnosis-related node;
a user interface module configured to generate a medical finding node based on user input;
an AI atlas module;
a volume meter module; and/or
a density meter module.

2. The method of claim 1, wherein, by way of the indicator, the respective ROI is displayed as an overlay to the original image; and, optionally,
wherein the indicator comprises, as a visual representation of the ROI, an outline, a marking and/or a filling of the ROI.

3. The method of claim 1 or 2, further comprising the user defining the ROI in the medical image.

4. The method of claim 1, further comprising:
displaying, in the first portion of the display, a plurality of indicators of different regions, wherein the added medical finding is associated with a group of regions, the group of regions comprising a set of the different regions selected by the user.

5. The method of one of claims 1 to 4, wherein at least one additional textual representation of a different medical finding associated with the region is displayed along with the textual representation (726; 764) of the medical finding in the second portion of the display, wherein the different medical finding is included in the medical report; and, optionally,
the method further comprising:
in response to the user designating one or more of the textual representation (726; 764) and the at least one additional textual representation, updating the medical report by removing, from the medical report, any of the medical findings represented by a textual representation not designated by the user.

6. The method of claim 5, further comprising:
using at least one of the designated textual representation and the medical finding represented by the designated textual representation as training data for training the AI module (706; 708; 740; 742; 744; 804; 812-826); and/or
using at least one of the added medical finding and the textual representation of the added medical finding as training data for training the AI module (706; 708; 740; 742; 744; 804; 812-826).

7. The method of one of claims 4 or 6, wherein at least one of steps (b) and (c) is repeated after updating the medical report.

8. The method of one of the preceding claims, wherein the medical image is obtained from a Picture Archiving and Communication System, PACS, or by loading an image based on a user command.

9. An apparatus (100) comprising at least one processor (102) and at least one memory (104), the at least one memory (104) containing instructions executable by the at least one processor (102) such that the apparatus (100) is operable to perform the method of one of claims 1 to 8.

10. A computer program product comprising program code portions for performing the method of one of claims 1 to 8 when the computer program product is executed on one or more processors (102).

11. The computer program product of claim 10, stored on one or more computer readable recording media.

## Patentansprüche

1. Verfahren zum Erzeugen eines medizinischen Berichts und zum Bereitstellen einer interaktiven Anzeige eines medizinischen Bildes (727) auf der Grundlage des medizinischen Berichts eines Patienten, wobei das Verfahren umfasst:
Erhalten eines medizinischen Bildes (cCT);
Erstellen eines medizinischen Bildknotens (702) in einem Graphen, der in einer Graphdatenbank gespeichert ist, unter Verwendung des medizinischen Bildes, wobei der medizinische Bildknoten (702) das medizinische Bild als Eigenschaft umfasst und wobei das medizinische Bild eines ist aus: einem Computertomographie-, CT-, Bild einem Magnetresonanz-, MR-, Bild, einem Röntgenbild oder einem Bild einer pathologischen Gewebeprobe;
automatisches Auslösen eines Anomalieerkennungsmoduls, das eingerichtet ist, um einen mit Bereich von Interesse, ROI, gekennzeichneten Knoten (724) in dem Graph zu definieren, wobei der mit ROI gekennzeichnete Knoten (724) mit dem medizinischen Bildknoten (702) verbunden ist;
Erhalten eines medizinischen Befunds für den mit ROI gekennzeichneten Knoten (724) unter Verwendung eines aus einer Programmmodul-Datenbank ausgewählten Programmmoduls;
Speichern des medizinischen Befunds als einen als lokaler Befund gekennzeichneten Knoten (716) des Graphs, wobei der medizinische Befund des als lokaler Befund gekennzeichneten Knotens (716) einem ROI, der durch den mit ROI gekennzeichneten Knoten des Graphs repräsentiert wird, über eine Kante zugeordnet ist, die den als lokaler Befund gekennzeichneten Knoten und den mit ROI gekennzeichneten Knoten verbindet;
Zuordnen des ROI-Knotens zu seinem entsprechenden medizinischen Befund-Knoten über eine Kante; und
Erstellen des medizinischen Berichts auf der Grundlage des Graphen, der eine textuelle Darstellung eines oder mehrerer der medizinischen Befunde umfasst;
wobei das Verfahren ferner umfasst:
(a) Anzeigen (202) eines medizinischen Bildes (727) des Patienten in einem ersten Abschnitt einer Anzeige;
wobei das Verfahren ferner (b) und/oder (c) umfasst:
(b) als Reaktion darauf, dass ein Benutzer einen Indikator (728) auswählt, der den mit ROI gekennzeichneten Knoten (724) des Graphen in dem angezeigten medizinischen Bild (727) visualisiert, wobei der ROI dem medizinischen Befund zugeordnet ist, der in dem medizinischen Bericht enthalten ist, Anzeigen (204) einer textuellen Darstellung (726; 764) des medizinischen Befunds, der als der als lokaler Befund gekennzeichnete Knoten (716) des Graphen gespeichert ist, in einem zweiten Abschnitt der Anzeige;
(c) Anzeigen (206) einer textuellen Darstellung (726; 764) des medizinischen Befunds, der als der als lokaler Befund gekennzeichnete Knoten (716) des Graphen gespeichert ist, in einem zweiten Abschnitt der Anzeige, wobei der medizinische Befund in dem medizinischen Bericht enthalten ist und dem ROI zugeordnet ist, der als der mit ROI gekennzeichnete Knoten (724) des Graphen in dem angezeigten medizinischen Bild (727) gespeichert ist, und als Reaktion darauf, dass ein Benutzer die angezeigte textuelle Darstellung (726; 764) auswählt, Anzeigen eines Indikators (728), der den ROI visualisiert, in dem ersten Abschnitt der Anzeige;
wobei das Verfahren ferner umfasst:
Aktualisieren des medizinischen Berichts (i) durch Hinzufügen eines medizinischen Befunds auf der Grundlage einer Benutzereingabe in den medizinischen Bericht, wobei der hinzugefügte medizinische Befund einem von dem Benutzer in dem angezeigten medizinischen Bild definierten Bereich zugeordnet ist, oder (ii) durch Aktualisieren des medizinischen Berichts durch Hinzufügen eines medizinischen Befunds in den medizinischen Bericht, wobei der medizinische Befund durch ein Programmmodul für künstliche Intelligenz, KI, bestimmt wird,
Definieren, basierend auf der Benutzereingabe, eines weiteren ROI des medizinischen Bildes als weiteren ROI-Knoten des Graphen und eines dem weiteren ROI zugeordneten medizinischen Befunds als weiteren lokalen Befund-Knoten des Graphen, wobei die Erstellung des weiteren lokalen Befund-Knotens eine Auswahl eines Programmmoduls auslöst, das eine mit dem weiteren lokalen Befund-Knoten übereinstimmende Eingabeanforderung aufweist;
Aktualisieren des medizinischen Berichts auf der Grundlage des weiteren ROI-Knotens und des weiteren lokalen Befund-Knotens;
wobei das Verfahren nach dem Hinzufügen des weiteren ROI-Knotens und des weiteren lokalen Befund-Knotens zu dem Graphen ferner umfasst:
Durchführen einer Abfrage der Programmmodul-Datenbank;
Erhalten eines oder mehrerer Programmmodule auf der Grundlage des aktuellen Zustands des Graphen;
Auslösen des einen oder der mehreren Programmmodule, wobei das eine oder die mehreren Programmmodule mindestens eines sind aus:
einem Bild-Viewer, der eingerichtet ist, um medizinische bildbezogene Daten anzuzeigen;
einem Anomalieerkennungsmodul, das eingerichtet ist, um einen Bereichsknoten zu erzeugen;
einem Lokalisierungsmodul, das eingerichtet ist, um einen anatomischen Lokalisationsknoten zu erzeugen;
einem Diagnosemodul, das eingerichtet ist, um einen diagnosebezogenen Knoten zu erzeugen;
einem Benutzeroberflächenmodul, das eingerichtet ist, um auf der Grundlage einer Benutzereingabe einen medizinischen Befund-Knoten zu erzeugen;
einem KI-Atlas-Modul;
einem Volumenmessmodul; und/oder
einem Dichtemessmodul.

2. Verfahren nach Anspruch 1, wobei der jeweilige ROI mittels des Indikators als Überlagerung auf dem Originalbild angezeigt wird; und, optional,
wobei der Indikator als visuelle Darstellung des ROIs einen Umriss, eine Markierung und/oder eine Füllung des ROIs umfasst.

3. Verfahren nach Anspruch 1 oder 2, ferner umfassend ein Definieren des ROIs in dem medizinischen Bild durch den Benutzer.

4. Verfahren nach Anspruch 1, ferner umfassend:
Anzeigen einer Vielzahl von Indikatoren von verschiedenen Bereichen in dem ersten Abschnitt der Anzeige, wobei der hinzugefügte medizinische Befund einer Gruppe von Bereichen zugeordnet ist, wobei die Gruppe von Bereichen einen Satz der verschiedenen, von dem Benutzer ausgewählten Bereichen umfasst.

5. Verfahren nach einem der Ansprüche 1 bis 4, wobei mindestens eine zusätzliche textuelle Darstellung eines anderen medizinischen Befunds, der dem Bereich zugeordnet ist, zusammen mit der textuellen Darstellung (726; 764) des medizinischen Befunds in dem zweiten Abschnitt der Anzeige angezeigt wird, wobei der andere medizinische Befund in dem medizinischen Bericht enthalten ist; und, optional,
wobei das Verfahren ferner umfasst:
als Reaktion darauf, dass der Benutzer eine oder mehrere der textuellen Darstellungen (726; 764) und die mindestens eine zusätzliche textuelle Darstellung bezeichnet, Aktualisieren des medizinischen Berichts durch Entfernen jeglicher medizinischen Befunde aus dem medizinischen Bericht, die durch eine von dem Benutzer nicht bezeichnete textuelle Darstellung repräsentiert werden.

6. Verfahren nach Anspruch 5, ferner umfassend:
Verwenden der bezeichneten textuellen Darstellung und/oder des durch die bezeichnete textuelle Darstellung repräsentierten medizinischen Befunds als Trainingsdaten zum Trainieren des KI-Moduls (706; 708; 740; 742; 744; 804; 812-826); und/oder
Verwenden der hinzugefügten medizinischen Befunde und/oder der textuellen Darstellung der hinzugefügten medizinischen Befunde als Trainingsdaten zum Trainieren des KI-Moduls (706; 708; 740; 742; 744; 804; 812-826).

7. Verfahren nach einem der Ansprüche 4 oder 6, wobei mindestens einer der Schritte (b) und (c) nach der Aktualisierung des medizinischen Berichts wiederholt wird.

8. Verfahren nach einem der vorhergehenden Ansprüche, wobei das medizinische Bild aus einem Bildarchivierungs- und Kommunikationssystem, PACS, oder durch Laden eines Bildes auf der Grundlage eines Benutzerbefehls erhalten wird.

9. Vorrichtung (100), die mindestens einen Prozessor (102) und mindestens einen Speicher (104) umfasst, wobei der mindestens eine Speicher (104) Befehle enthält, die von dem mindestens einen Prozessor (102) ausgeführt werden können, so dass die Vorrichtung (100) betreibbar ist, das Verfahren gemäß einem der Ansprüche 1 bis 8 durchzuführen.

10. Computerprogrammprodukt, das Programmcodeabschnitte zur Ausführung des Verfahrens gemäß einem der Ansprüche 1 bis 8 umfasst, wenn das Computerprogrammprodukt auf einem oder mehreren Prozessoren (102) ausgeführt wird.

11. Computerprogrammprodukt nach Anspruch 10, das auf einem oder mehreren computerlesbaren Speichermedien gespeichert ist.

## Revendications

1. Procédé pour générer un rapport médical et fournir un affichage interactif d'une image médicale (727) sur la base du rapport médical d'un patient, le procédé comprenant :
l'obtention d'une image médicale (cCT) ;
la création d'un nœud d'image médicale (702) dans un graphe stocké dans une base de données de graphes à l'aide de l'image médicale, dans lequel le nœud d'image médicale (702) comprend l'image médicale en tant que propriété, et dans lequel l'image médicale est une image parmi : une image de tomodensitométrie, CT, une image de résonance magnétique, RM, une image radiographique ou une image d'échantillon de tissu pathologique ;
le déclenchement automatique d'un module de détection d'anomalies configuré pour définir un nœud étiqueté de région d'intérêt, ROI, (724) dans le graphe, dans lequel le nœud étiqueté de ROI (724) est relié au nœud d'image médicale (702) ;
l'obtention, pour le nœud étiqueté de ROI (724), d'une constatation médicale au moyen d'un module de programme sélectionné dans une base de données de modules de programme,
le stockage de la constatation médicale sous forme de nœud étiqueté de constatation locale (716) du graphe, dans lequel la constatation médicale du nœud étiqueté de constatation locale (716) est associée à une ROI représentée par le nœud étiqueté de ROI du graphe par une arête reliant le nœud étiqueté de constatation locale et le nœud étiqueté de ROI ;
l'association du nœud de ROI avec son nœud de constatation médicale correspondant au moyen d'une arête ; et
la génération du rapport médical sur la base du graphe comprenant une représentation textuelle d'une ou plusieurs des constatations médicales ;
dans lequel le procédé comprend en outre :
(a) l'affichage (202) d'une image médicale (727) du patient dans une première partie d'un affichage ;
le procédé comprenant en outre au moins l'un de (b) ou (c) :
(b) en réponse à la sélection par un utilisateur d'un indicateur (728) visualisant la ROI stockée en tant que nœud étiqueté de ROI (724) du graphe dans l'image médicale affichée (727), la ROI étant associée à la constatation médicale incluse dans le rapport médical, l'affichage (204) d'une représentation textuelle (726 ; 764) de la constatation médicale stockée en tant que nœud étiqueté de constatation locale (716) du graphe dans une deuxième partie de l'affichage ;
(c) l'affichage (204) d'une représentation textuelle (726 ; 764) de la constatation médicale stockée en tant que nœud étiqueté de constatation locale (716) du graphe dans une deuxième partie de l'affichage, la constatation médicale incluse dans le rapport médical et associée à la ROI stockée en tant que nœud étiqueté de ROI (724) du graphe dans l'image médicale affichée (727), et, en réponse à la sélection par un utilisateur de la représentation textuelle affichée (726 ; 764), l'affichage, dans la première partie de l'affichage, d'un indicateur (728) visualisant la ROI ;
dans lequel le procédé comprend en outre :
la mise à jour du rapport médical (i) par ajout, dans le rapport médical, d'une constatation médicale basée sur l'entrée de l'utilisateur, dans lequel la constatation médicale ajoutée est associée à une région définie par l'utilisateur dans l'image médicale affichée, ou (ii) par mise à jour du rapport médical en ajoutant, dans le rapport médical, une constatation médicale, la constatation médicale étant déterminée par un module de programme d'intelligence artificielle, IA,
la définition, sur la base de l'entrée de l'utilisateur, d'une ROI supplémentaire de l'image médicale en tant que nœud de ROI supplémentaire du graphe et d'une constatation médicale ajoutée, associée à la ROI supplémentaire, en tant que nœud de constatation locale supplémentaire du graphe, dans lequel la création du nœud de constatation locale supplémentaire déclenche une sélection d'un module de programme dont les exigences d'entrée correspondent au nœud de constatation locale supplémentaire ;
la mise à jour du rapport médical sur la base du nœud de ROI supplémentaire et du nœud de constatation locale supplémentaire ;
dans lequel, lors de l'ajout du nœud de ROI supplémentaire et du nœud de constatation locale supplémentaire au graphe, le procédé comprend en outre :
la réalisation d'une interrogation de la base de données de modules de programme ;
l'obtention d'un ou plusieurs modules de programme sur la base de l'état actuel du graphe ;
le déclenchement desdits un ou plusieurs modules de programme, dans lequel lesdits un ou plusieurs modules de programme sont au moins l'un parmi :
un visualiseur d'images configuré pour afficher des données relatives à une image médicale ;
un module de détection d'anomalies configuré pour générer un nœud de région ;
un module de localisation configuré pour générer un nœud de localisation anatomique ;
un module de diagnostic configuré pour générer un nœud associé à un diagnostic ;
un module d'interface utilisateur configuré pour générer un nœud de constatation médicale sur la base de l'entrée de l'utilisateur ;
un module d'atlas d'IA ;
un module de mesure de volume ; et/ou
un module de mesure de densité.

2. Procédé selon la revendication 1, dans lequel, au moyen de l'indicateur, la ROI respective est affichée en superposition sur l'image originale ; et, éventuellement,
dans lequel l'indicateur comprend, en tant que représentation visuelle de la ROI, un contour, un marquage et/ou un remplissage de la ROI.

3. Procédé selon la revendication 1 ou 2, comprenant en outre la définition, par l'utilisateur, de la ROI dans l'image médicale.

4. Procédé selon la revendication 1, comprenant en outre :
l'affichage, dans la première partie de l'affichage, d'une pluralité d'indicateurs de régions différentes, dans lequel la constatation médicale ajoutée est associée à un groupe de régions, le groupe de régions comprenant un ensemble des régions différentes sélectionnées par l'utilisateur.

5. Procédé selon l'une des revendications 1 à 4, dans lequel au moins une représentation textuelle supplémentaire d'une constatation médicale différente associée à la région est affichée avec la représentation textuelle (726 ; 764) de la constatation médicale dans la deuxième partie de l'affichage, dans lequel la constatation médicale différente est incluse dans le rapport médical ; et, éventuellement,
le procédé comprenant en outre :
en réponse à la désignation, par l'utilisateur, d'une ou plusieurs parmi la représentation textuelle (726 ; 764) et ladite au moins une représentation textuelle supplémentaire, la mise à jour du rapport médical par la suppression, dudit rapport médical, de toute constatation médicale représentée par une représentation textuelle non désignée par l'utilisateur.

6. Procédé selon la revendication 5, comprenant en outre :
l'utilisation d'au moins l'une de la représentation textuelle désignée et de la constatation médicale représentée par la représentation textuelle désignée en tant que données d'apprentissage pour entraîner le module d'IA (706 ; 708 ; 740 ; 742 ; 744 ; 804 ; 812 à 826) ; et/ou
l'utilisation d'au moins l'une de la constatation médicale ajoutée et de la représentation textuelle de la constatation médicale ajoutée en tant que données d'apprentissage pour entraîner le module d'IA (706 ; 708 ; 740 ; 742 ; 744 ; 804 ; 812 à 826).

7. Procédé selon l'une des revendications 4 ou 6, dans lequel au moins l'une des étapes (b) et (c) est répétée après la mise à jour du rapport médical.

8. Procédé selon l'une des revendications précédentes, dans lequel l'image médicale est obtenue à partir d'un système d'archivage et de communication d'images, PACS, ou par le chargement d'une image sur la base d'une commande de l'utilisateur.

9. Appareil (100) comprenant au moins un processeur (102) et au moins une mémoire (104), ladite au moins une mémoire (104) contenant des instructions exécutables par ledit au moins un processeur (102) de telle sorte que l'appareil (100) puisse fonctionner pour mettre en œuvre le procédé selon l'une des revendications 1 à 8.

10. Produit programme informatique comprenant des parties de code de programme pour mettre en œuvre le procédé selon l'une des revendications 1 à 8 lorsque le produit programme informatique est exécuté sur un ou plusieurs processeurs (102).

11. Produit programme informatique selon la revendication 10, stocké sur un ou plusieurs supports d'enregistrement lisibles par ordinateur.
